# EUROPÄISCHE PATENTSCHRIFT

(11) **EP 0 628 553 B1**
(45) Veröffentlichungstag und Bekanntmachung des Hinweises auf die Patenterteilung: **02.09.1998**
(21) Anmeldenummer: 94108037.6
(22) Anmeldetag: 25.05.1994
(51) Int. Cl.: C07D 317/36, C07D 317/38

(54) **Reaktor und damit durchzuführendes kontinuierliches Verfahren zur Herstellung von Ethylenglykolcarbonat bzw. Propylenglykolcarbonat**
Reactor and continuously performed with it process for preparation of ethylene glycol carbonate or propylene glycol carbonate
Réacteur et procédé de préparation en continu de carbonate d'éthylène glycol ou de propylène glycol réalisé dans ce réacteur

(30) Priorität: 07.06.1993 DE 4318893
(43) Veröffentlichungstag der Anmeldung: 14.12.1994
(73) Patentinhaber: BAYER AG, 51368 Leverkusen (DE)
(72) Erfinder: Wagner, Paul, Dr., D-40597 Düsseldorf (DE); Mendoza-Frohn, Christine, Dr., D-40699 Erkrath 1 (DE); Buysch, Hans-Josef, Dr., D-47809 Krefeld (DE)

(56) Entgegenhaltungen:
- EP-A- 0 546 428
- SU-A- 172 342
- PATENT ABSTRACTS OF JAPAN, Band 6, Nr. 197 (C-128)(1075), 06 Oktober 1982
- CHEMICAL ABSTRACTS, Band 99, Nr. 18, 1983, Columbus, OH (US); Seite 102, AN 141920f

## Beschreibung

Die vorliegende Erfindung betrifft einen Blasensäulenreaktor und ein damit durchzuführendes Verfahren zur Herstellung von Ethylenglykolcarbonat (EGC) bzw. Propylenglykolcarbonat (PGC) durch Umsetzung von CO₂ mit Ethylenoxid (EOX) bzw. Propylenoxid (POX) in bereits vorhandenem EGC bzw. PGC als Reaktionsmedium. Die Durchführung des genannten Verfahrens im erfindungsgemäß bereitgestellten Blasensäulenreaktor erfolgt besonders schonend für die Reaktionspartner und den einzusetzenden Katalysator, da Nebenreaktionen des eingesetzten EOX bzw. POX weitgehend minimiert werden können. Dies erfolgt durch eine hohe Selektivität der Umsetzung von EOX bzw. POX mit CO₂. Hieraus ergibt sich ein energie-und materialsparendes Verfahren. Es wird in bevorzugter Weise adiabatisch betrieben.

Die Herstellung von EGC bzw. PGC aus CO₂ und EOX bzw. POX ist lange bekannt (z.B. SU-A-172342). Viele Publikationen auf diesem Gebiet beziehen sich vornehmlich auf den Einsatz bestimmter Katalysatoren. Eine ausführliche Darstellung hierzu findet sich in Fette, Seifen, Anstrichmittel 73 (1971), 396. Weitere Publikationen sind beispielsweise Ind. Eng Chem. 50 (1958), 767, Chem. Ing. Techn. 43 (1971), 903, und DE-OS 28 55 232 (äquivalent zu US 4.314.945).

Das in Ind. Eng. Chem. (loc. cit.) beschriebene Verfahren zur Herstellung von EGC bzw. PGC in Gegenwart von Tetraethylammoniumbromid als Katalysator wird bei 180°C und 100 bar in einem Umpumpreaktor durchgeführt. Dieser Umpumpreaktor besteht aus einem von den Reaktanden und dem als Reaktionsmedium dienenden Carbonat abwärts durchströmten Rohr, das als Reaktionsteil dient und aus einem externen Umlauf, in dem sich ein Wärmetauscher zur Abkühlung der umgepumpten Flüssigkeit auf 50°C befindet. Die Einspeisung des EOX bzw. POX und des Katalysators, gelöst in dem entsprechenden Carbonat, erfolgt am oberen Ende des Reaktionsrohres, in dem sich eine dünne Schicht mit keramischen Ringen als Mischungszone befindet. Die weitere Durchmischung der Reaktanden im Reaktor wird durch das Umpumpen bewerkstelligt. CO₂ wird am unteren Ende des Reaktionsrohres mit einem 6- bis 7 %igen Überschuß, bezogen auf das Alkylenoxid, zudosiert. Der Produktstrom aus dem Umpumpreaktor wird durch einen weiteren Rohrreaktor, der als Nachreaktor zur Vervollständigung des Umsatzes des Ethylenoxids dient und ebenfalls bei 180°C und 100 bar betrieben wird, geführt. Dieser beschriebene Rohrreaktor hat den Nachteil, daß zusätzlich zu den ohnehin drastischen Bedingungen Temperaturspitzen im Reaktionsteil auftreten können, die sich produktschädigend auswirken. Diese Temperaturspitzen können ohne besondere Vorsichtsmaßnahmen 200°C überschreiten. Um diesen Temperaturspitzen entgegenzuwirken, ist ein hoher Grad an Rückvermischung vorgesehen. Die örtlich benachbarte Dosierung von Alkylenoxid und Katalysator bei gleichzeitig getrennter Zuspeisung des Reaktionspartners CO₂ führt zu hohen lokalen Überschüssen an Alkylenoxid. Diese Dosierform und die Rückvermischung im Reaktionsteil begünstigen die Nebenreaktionen des Alkylenoxids und stellen im Zusammenhang mit den beschriebenen Temperaturspitzen weitere Nachteile dieser Reaktionsführung dar. Aufgrund der hohen Exothermie der Umsetzung von CO₂ mit EOX bzw. POX muß der Umsatz im ersten Reaktor begrenzt und in einem Nachreaktor vervollständigt werden.

In dem in Fette, Seifen (loc. cit.) und Chem. Ing. Techn. (loc. cit.) beschriebenen Reaktor, der aus einem einfachen Rohr ohne Einbauten und ohne Produktkreislauf besteht, werden CO₂ und EOX bzw. POX bei 80 bar und 190 bis 200°C in Gegenwart eines im zu bildenden Carbonat gelösten Katalysator miteinander umgesetzt und die Reaktionswärme mit Hilfe eines im Gegenstrom zirkulierenden Wärmeträgers abgeführt, der wiederum mit Wasser gekühlt wird. Auch bei dieser Art der Reaktionsführung erhält man Spitzentemperaturen von bis zu 220°C im Reaktor, die produktschädigend wirken, worauf in Fette, Seifen (loc. cit.) ausdrücklich verwiesen wird; solche Temperaturspitzen sind insbesondere bei einer technischen Großanlage schwer zu beherrschen. Die gesamte Reaktionswärme wird bei dieser Reaktionsführung ungenutzt abgeführt. Eine Nutzung wäre in besserer Weise durch eine adiabatische Reaktionsführung zu erzielen, bei der die gesamte freiwerdende Reaktionswärme durch das Reaktionsgemisch selbst aufgenommen wird. Dies jedoch führt bei exothermen Reaktionen stets zu einer Erhöhung der Temperatur des Reaktionsgemisches, auf deren schädlichen Einfluß die genannten Publikationen hinweisen. Daher wird eine adiabatische Reaktionsführung in Fette, Seifen (loc. cit.) als technisch nicht realisierbar angesehen. Wie auch im weiter oben beschriebenen Verfahren muß der Umsatz in einem Nachreaktor vervollständigt werden. Auch bei dem beschriebenen Reaktor ohne Einbauten und ohne äußeren Produktkreislauf können aufgrund lokaler Alkylenoxid-Überkonzentrationen gegenüber CO₂ Nebenreaktionen des Alkylenoxids stattfinden, die zu einer Verringerung der Selektivität der Reaktion und damit zu einer Verringerung der Ausbeute führen.

In der genannten DE-OS '232 werden als Reaktor für die Herstellung von Alkylencarbonaten aus Alkylenoxiden und CO₂ unterschiedliche Kombinationen von Strömungsrohren und Umpumpreaktoren beschrieben, die bei 10 bis 50 bar und 100 bis maximal 200°C betrieben werden. Das zu bildende Carbonat dient in allen Reaktorteilen als Reaktionsmedium und stellt dort jeweils 85 bis 99.6 Gew.-% aller im Reaktor vorhandenen Stoffe dar. Die Edukte CO₂ und Alkylenoxid werden in die erste Reaktionszone einer solchen Kombination gemeinsam mit dem in dem zu bildenden Carbonat gelösten Katalysator dosiert. Dabei wird CO₂ nur im leichten molaren Überschuß gegenüber dem Katalysator zugespeist. Als Bauteile für die eingesetzten Reaktorkombinationen werden Rührkessel mit äußerem Umlauf zur Produktkühlung und Strömungsrohre eingesetzt. Diesen Reaktorbauteilen ist unabhängig von ihrer Anzahl und Reihenfolge innerhalb der Kombination gemeinsam, daß in ihnen jeweils nur ein Teilumsatz des Alkylenoxids stattfinden kann, da sonst das Wärmeproblem der stark exothermen Umsetzung nicht beherrscht werden kann. Nur durch eine solche aufwendige Verschaltung der Reaktoren mit Kühlern und Durchführung von Teilumsätzen kann insgesamt ein Umsatz des Alkylenoxids von etwa 99,5 % und eine Carbonatselektivität von etwa 99 % erhalten werden.

Es bestand daher der Wunsch, einen einfachen und beliebig vergrößerbaren Reaktor für die vollständige und selektive Umsetzung von Alkylenoxiden und CO₂ zu den zugehörigen Alkylencarbonaten zur Verfügung zu haben, der auf Nachreaktoren, auf aufwendige Kühlsysteme oder eine komplexe Kopplung von Reaktorbauteilen und Kühlem verzichten kann. In einem solchen Reaktor sollte weiterhin die Bildung schädlicher Temperaturspitzen vermieden werden können. Ein ergänzender Wunsch besteht in der weitestgehenden Nutzung der exothermen Wärmetönung der Reaktion, beispielsweise im Rahmen der Aufarbeitung des Reaktionsproduktes oder zur Erzeugung von Prozeßdampf.

Die Erfindung betrifft einen Reaktor zur kontinuierlichen Herstellung von Ethylenglykolcarbonat (EGC) bzw. Propylenglykolcarbonat (PGC) aus CO₂ und Ethylenoxid (EOX) bzw. Propylenoxid (POX) in bereits vorliegendem EGC bzw. PGC als Reaktionsmedium in Form einer Blasensäule, gekennzeichnet durch a) einen Schlankheitsgrad als Verhältnis von Höhe zu Durchmesser von 2-80, bevorzugt 2 bis 50, besonders bevorzugt 5 bis 30, b) einen Zulauf für das als Reaktionsmedium dienende, bereits vorliegende EGC bzw. PGC am unteren Ende der Blasensäule, c) eine oder mehrere Dosierstellen für die Edukte CO₂ und EOX bzw. POX oberhalb des Zulaufs b), d) einen Ablauf für das Reaktionsgemisch am oberen Ende der Blasensäule, e) eine an d) anschließende Aufteilungseinrichtung für das ablaufende Reaktionsgemisch zur Aufteilung in zu entnehmendes und aufzuarbeitendes Reaktionsgemisch (11) und in zu recyclisierendes Reaktionsgemisch (10) und f) eine Rückführangsleitung (10) und (3) zum Zulauf b) für das zu recyclisierende Reaktionsgemisch.

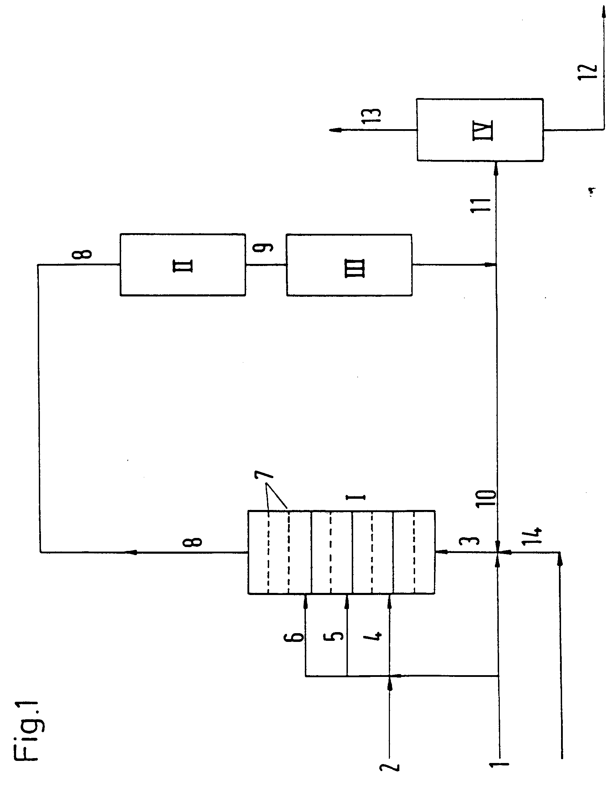

Die in Klammern gesetzten Bezugszahlen beziehen sich auf die beigefügte Fig. 1.

Die Erfindung betrifft weiterhin ein Verfahren zur kontinuierlichen Herstellung von Ethylenglykolcarbonat (EGC) bzw. Propylenglykolcarbonat (PGC) durch Umsetzung von Ethylenoxid (EOX) bzw. Propylenoxid (POX) mit 1,01 bis 1,5 Mol, bevorzugt 1,01 bis 1,4 Mol, besonders bevorzugt 1,01 bis 1,35 Mol CO₂ pro Mol Alkylenoxid im Temperaturbereich von 100 bis 200°C, bevorzugt 100 bis 190°C, besonders bevorzugt 110 bis 180°C und im Druckbereich von 5 bis 200 bar, bevorzugt 5 bis 80 bar, besonders bevorzugt 8 bis 60 bar im zu bildenden EGC bzw. PGC als Reaktionsmedium in einer Menge, die das 7- bis 350-fache des neu gebildeten EGC bzw. PGC beträgt, das dadurch gekennzeichnet ist, daß zur Umsetzung ein Blasensäulenreaktor, wie oben beschrieben, verwendet wird.

Die Ausgangsprodukte EOX bzw. POX und CO₂ werden in der Regel in einer Reinheit von 99 % und höher eingesetzt. Es ist jedoch gleichfalls möglich, die Ausgangsstoffe in geringerer Reinheit einzusetzen, wenn der Rest zu 100 % aus inerten Stoffen, beispielsweise Kohlenwasserstoffen, Kohlenmonoxid oder Stickstoff, besteht. Dies gilt besonders für CO₂, das aus verschiedenen Quellen stammen kann, beispielsweise aus natürlichen Quellen oder aus Anlagen zur Erzeugung von Wassergas, Kohlenmonoxid oder Reformern, und dementsprechend von geringerer Reinheit ist. Solche Inertgase sind jedoch zweckmäßigerweise in einem Anteil von nicht mehr als 10 Vol.-%, bevorzugt nicht mehr als 5 Vol.-%, besonders bevorzugt nicht mehr als 3 Vol.-% anwesend.

Als Katalysatoren können praktisch alle bisher vorgeschlagenen verwendet werden, wie Alkali- und Erdalkalibromide und -iodide, Guanidine und deren Hydrobromide oder Hydroiodide, Tetraalkylammoniumbromide und -iodide, Phosphoniumbromide und -iodide, Pyridiniumhalogenide, Sulfonium-, Stibonium- und Arsoniumhalogenide, Zink- und Bleihalogenide, Alkylzinnverbindungen oder Gemische aus Alkalihalogeniden mit Halogeniden zweiwertiger Metallionen. Diese Katalysatoren sind dem Fachmann bekannt und nicht Gegenstand der Erfindung. In bevorzugter Weise werden als Katalysatoren eingesetzt: Alkalibromide und -iodide, Tetraalkylammoniumbromide und -iodide, Phosphoniumhalogenide, Guanidiniumhalogenide und Gemische aus Alkalihalogeniden mit Halogeniden zweiwertiger Metalle.

Der erfindungsgemäße Reaktor und das damit durchzuführende erfindungsgemäße Verfahren werden im folgenden unter Hinweis auf die beigefügte Fig. 1 beschrieben. Der Reaktor besteht im wesentlichen aus einer Blasensäule (I), die mit einem äußeren Umlauf versehen ist. Diese Blasensäule kann für die bevorzugte adiabatische Verfahrensdurchführung in geeigneter Weise isoliert sein. (I) ist mit einer oder in bevorzugter Weise mit mehreren Dosierstellen für die Edukte CO₂ (1) und EOX bzw. POX (2) in das als Reaktionsmedium dienende und bei (3) eindosierte EGC bzw. PGC hinein ausgestattet. In Fig. 1 sind beispielsweise drei Dosierstellen (4), (5) und (6) dargestellt; weitere Dosierstellen können in analoger Weise vorgesehen werden. Die größere Anzahl Dosierstellen, verglichen mit nur einer Dosierstelle, trägt zur Vermeidung örtlicher Überkonzentrationen an EOX bzw. POX und zur Vermeidung örtlicher Temperaturspitzen im Reaktor bei.

Weiterhin ist es möglich, CO₂ auch schon unterhalb der Blasensäule in das über (10) und (3) zulaufende EGC bzw. PGC einzuspeisen. Die Ausgangsstoffe werden in üblicher und bevorzugter Weise gasförmig zudosiert. (I) wird von den eingespeisten Ausgangsmaterialien und dem Reaktionsmedium im Gleichstrom von unten nach oben durchströmt. Zur weiteren Förderung der Reaktion kann (I) in bevorzugter Weise mit Einbauten (7) versehen sein, wodurch sich die Bauform einer kaskadierten Blasensäule ergibt.

Einbauten zur Dosierung der Ausgangsstoffe und Einbauten zur Gewährleistung einer fortlaufenden guten Verteilung und intensiven Durchmischung aller beteiligten Stoffe des Reaktionsgemisches an allen Stellen im Reaktor sind beispielsweise Lochplatten, Loch- und Prallplattenböden, Rohrverteiler (Einsteck- und Ringverteiler), Zweistoffdüsen, Strahldüsen, Düsenböden, weitere Düsen, beispielsweise gemäß DE-OS 37 36 988 bzw. DE-OS 37 44 001, Begasungsböden, Sintermetallfritten, geschlossene Gasverteilerböden mit Durchtrittsöffnungen für das Reaktionsmedium, rotierende Begasungsapparate, Impingment Aerator-Elemente, beispielsweise nach Perry's Chemical Engineers' Handbook 1984, Seiten 18.57 bis 18.70, Mischerelemente, Blecheinbauten zur Erhöhung der Turbulenz, Segment- und Kreisringumlenkbleche. Solche Einbauten können auch in Kombination miteinander eingesetzt werden. Insbesondere sollten solche Einbauten neben einer guten Durchmischung der Strömung (Makrovermischung) und der feinen Gasverteilung einen möglichst großen Anteil hochfrequenter Turbulenzelemente im Dissipationsbereich erzeugen. Bevorzugte Einbauten sind Lochböden, Rohrverteiler, Zweistoffdüsen, Strahldüsen, weitere Düsen, beispielsweise nach DE-OS 37 36 988 bzw. DE-OS 37 44 001, geschlossene Gasverteiler, Impingment Aerator-Elemente und Mischerelemente.

Zur Vermeidung unerwünschter Temperaturspitzen und unerwünschter örtlicher Überkonzentrationen wird im gesamten, aus Blasensäule und externem Umlauf bestehenden Reaktor eine intensive Durchmischung aufrechterhalten.

(I) besitzt einen Schlankheitsgrad als Verhältnis von Höhe zu Durchmesser von 2 bis 50, bevorzugt von 5 bis 30; hierdurch wird der Grad der Rückvermischung innerhalb der Blasensäule zurückgedrängt. Diese Herabsetzung der Rückvermischung der Blasensäule wird auch durch die oben beschriebenen Einbauten unterstützt.

Ein geringer Grad an Rückvermischung ist jedoch andererseits wünschenswert, weil schon bei einem einmaligen Durchgang der Ausgangsstoffe durch die Blasensäule bei ausreichender, von der Art und Konzentration des Katalysators und von den Bedingungen sowie von der Reaktionsfuhrung abhängiger, theoretisch berechenbarer und durch Vorversuche zu verifizierender mittlerer Verweilzeit ein praktisch vollständiger Umsatz des Alkylenoxids erreicht werden soll.

Dieser geringe Grad an Rückvermischung begünstigt weiterhin die Selektivität der Reaktion des Alkylenoxids mit dem CO₂ zum gewünschten Carbonat, da die Nebenreaktionen der Alkylenoxide zu polykondensierten Aldehyden eine Folge von Rückvermischung und schlechter Durchmischung sind.

Für den bevorzugten Fall einer kaskadierten Bauweise für den Blasensäulenreaktor beträgt der Abstand zwischen je zwei aufeinanderfolgenden Einbauten zur Gasverteilung das 0,1- bis 10-fache, bevorzugt das 0,3- bis 8-fache des Blasensäulendurchmessers. Dies gilt in gleicher Weise für den weiterhin bevorzugten Fall mehrerer Dosierstellen für die Ausgangsmaterialien. Bei diesen Einbauten wird der freie Begasungsquerschnitt so bemessen, daß eine Gasgeschwindigkeit von 0,1 bis 15 m/s, bevorzugt 0,2 bis 12 m/s beträgt. Hierzu wird der Durchmesser der Bohrungen in den Einbauten auf Werte von 0,2 bis 20 mm, bevorzugt 0.2 bis 10 mm, besonders bevorzugt 0,5 bis 8 mm festgelegt.

Unter Berücksichtigung der fortlaufenden Absorption und Reaktion der gasförmigen Ausgangsmaterialien in der Blasensäule nimmt das Gasvolumen in Strömungsrichtung ab. In bevorzugter Weise kann demnach der freie Durchtrittsquerschnitt der verwendeten Einbauten zur erneuten Gasverteilung von unten nach oben in der Blasensäule abnehmen.

Der erfindunsgemäße Reaktor wird im Rahmen des damit durchzuführenden erfindungsgemäßen Verfahrens im Temperaturbereich von 100 bis 200°C, bevorzugt 100 bis 190°C, besonders bevorzugt 110 bis 180°C und bei einem Druck von 2 bis 200 bar, bevorzugt 5 bis 80 bar, besonders bevorzugt 8 bis 60 bar betrieben; bei Temperaturen im oberen Teil des angegebenen Bereiches werden auch Drücke im oberen Teil des angegebenen Bereiches benutzt und umgekehrt. Die adiabatische Temperatursteigerung im erfindungsgemäßen Verfahren wird auf 2 bis 80°C begrenzt. Die Eingangstemperatur in (I) wird hierbei so gewählt, daß auch bei voller Ausnutzung der gewählten adiabatischen Temperatursteigerung die Obergrenze des angegebenen Temperaturbereiches für den gesamten Reaktor nicht überschritten wird.

An allen Stellen des Reaktors wird ein CO₂-Überschuß über das EOX bzw. das POX aufrechterhalten. Dieser Überschuß beträgt 1,01 bis 1,5 Mol, bevorzugt 1,01 bis 1,4 Mol, besonders bevorzugt 1,01 bis 1,35 Mol.

EGC bzw. PGC als Reaktionsmedium liegt in der Blasensäule (I) stets in großem Überschuß über das neu gebildete EGC bzw. PGC vor. So läuft der Blasensäule pro Zeiteinheit das 7- bis 350-fache des in dieser Zeiteinheit gebildeten EGC bzw. PGC als Umlauf-Carbonat, das auch den Katalysator enthält zu. Dieser große Überschuß wird weiterhin dadurch aufrechterhalten, daß 80 bis 98 Gew.-%, bevorzugt 85 bis 97 Gew.-% des gesamten Reaktionsgemisches über die Rückführungsleitung an den Eingang der Blasensäule zurückgeführt werden; lediglich der Rest zu 100 % an Reaktionsgemisch wird abgezogen und auf reines EGC bzw. PGC aufgearbeitet.

Das erfindungsgemäße Verfahren ist in seiner bevorzugten Form durch eine adiabatische Temperaturführung gekennzeichnet. Hierbei wird das Reaktionsgemisch durch die exotherme Wärmetönung um 2 bis 80°C erwärmt. Diese fühlbare, dem Reaktionsgemisch innewohnende Wärme kann auf verschiedene Weise genutzt werden, wobei eine Abkühlung des Reaktionsgemisches um wiederum 2 bis 80°C eintritt. Die wichtigste dieser Nutzungsmöglichkeiten ist die zur Aufarbeitung des Reaktionsgemisches zum reinen EGC bzw. PGC. Für den Fall, daß die erreichte fühlbare Wärme des Reaktionsgemisches sehr groß ist, kann darüber hinaus noch Heizdampf erzeugt werden, der für andere Zwecke als die des erfindungsgemäßen Verfahrens eingesetzt werden kann.

Der erfindungsgemäße Reaktor ist durch eine oder mehrere Dosierstellen für die gasförmigen Ausgangsstoffe CO₂ und EOX bzw. POX gekennzeichnet. Für den Fall nur einer Dosierstelle werden dort alle gasförmigen Ausgangsmaterialien gemeinsam zugesetzt. Für den Fall mehrerer Dosierstellen können grundsätzlich CO₂ und EOX bzw. POX getrennt zugesetzt werden, wobei in bevorzugter Weise der Zusatz von CO₂ vor dem des EOX bzw. POX erfolgt. In einer bevorzugten Ausführungsförm des erfindungsgemäßen Reaktors sind jedoch alle Dosierstellen so angeschlossen, daß durch sie eine gemeinsame Dosierung von CO₂ und Alkylenoxid erfolgt. In einer weiteren bevorzugten Ausführungsform wird zusätzlich zu dem Anschluß der Dosierstellen für die gemeinsame Dosierung von CO₂ und Alkylenoxid eine Vorabeinspeisung von CO₂ in die Rückführungsleitung (10) und (3) vorgesehen. Diese Vorabeinspeisung von CO₂ umfaßt in weiterhin bevorzugter Weise den molaren Überschuß des CO₂ über das Alkylenoxid, wobei über die weiter obenliegenden Dosierstellen CO₂ und Alkylenoxid als äquimolares Gemisch eingespeist wird. Durch diese Ausführungsform des Reaktors wird gewährleistet daß CO₂ stets in, Überschuß über das Alkylenoxid und zwar an allen Stellen des Reaktors vorliegt. Die Zahl der Dosierstellen für gemeinsames Einspeisen von CO₂ und Alkylenoxid beträgt 1 bis 10, bevorzugt 2 bis 5. Die Zahl der zusätzlichen Einbauten zur Verteilung und Durchmischung aller beteiligten Stoffe, wie oben beschrieben, beträgt 0 bis 50, bevorzugt 3 bis 30; der Wert Null stellt den Fall dar, bei dem nur Dosierstellen/einbauten für CO₂ und Alkylenoxid vorhanden sind.

Das aus (I) ablaufende Reaktionsgemisch wird über einen externen Umlauf (8), (9), (10) und (3) zum größten Teil wieder nach (I) zurückgeführt. Fig. 1 zeigt im Verlaufe dieser Rückführungsleitung Wärmetauscheinrichtungen (II) und (III). Für den Fall, daß die dem Reaktionsgemisch innewohnende fühlbare Wärme für die Aufarbeitung des Reaktionsgemisches genutzt wird und insbesondere für den Fall, daß diese Aufarbeitung auf destillativem Wege erfolgt, kann (II) der Heizmantel eines Verdampfers für das aufzuarbeitende Reaktionsgemisch, beispielsweise eines Schnellverdampfers, sein. (III) ist in Fig. 1 beispielhaft als ein weiterer Wärmeaustauscher angegeben, in welchem etwa Heizdampf erzeugt wird. Die Anordnung von (II) und (III) ist in Fig. 1 lediglich beispielhaft angegeben und kann auch in umgekehrter Reihenfolge vorgenommen werden. Ebenso kann an die Stelle des heizdampferzeugenden Wärmetauschers (III) auch ein zu beheizender Reaktor eines völlig anderen Verfahrens treten, dessen Reaktion endotherm verläuft. Das Reaktionsgemisch wird im Beispiel der Fig. 1 nach Durchtritt durch (III) in die Teilströme (10) und (11) aufgeteilt. Der Teilstrom (10) umfaßt in der oben bereits beschriebenen Weise 80 bis 98 Gew.-% des gesamten Ablaufs der Blasensäule (I); der Teilstrom (11) stellt den Rest zu 100 % dar. Selbstverständlich ist es grundsätzlich möglich, die Aufteilung in die Teilströme (10) und (11) bereits zwischen den Wärmetauschern (II) und (III) oder bereits vor dein Wärmetauscher (II) vorzunehmen.

Während (10) zum Eingang der Blasensäule rückgeführt wird, wird der Teilstrom (11) der Aufarbeitung zugeführt. Hierzu gelangt (11) zunächst in einen Entspannungsbehälter (IV), worin eine Trennung in Flüssigkeit (12) und Gas (13) erfolgt. Die Gasphase (13) enthält überschüssiges CO₂ und gegebenenfalls Spuren von nicht umgesetztem Alkylenoxid sowie evtl. mit dem CO₂ und dem Alkylenoxid eingebrachte Inertgase. Je nach dem Anteil an Inertgasen kann ein Teilstrom von (13) wieder in den Reaktor zurückgeführt werden, während der Rest einer Abgasentsorgung zugeführt wird. Es ist selbstverständlich, daß bei höheren Inertgasanteilen ein höherer Teil von (13) zur Abgasentsorgung ausgeschleust wird und umgekehrt; einfache analytische Bestimmungen und Berechnungen sowie Vorversuche ergeben für den Fachmann ohne Schwierigkeiten das Optimum der Aufteilung von (13). Die in (IV) abgetrennte Flüssigkeit (12) wird der Reinproduktgewinnung des EGC bzw. PGC, beispielsweise durch Destillation zugeführt.

Der Katalysator für das erfindungsgemäße Verfahren wird über (14) in die Rückführungsleitung (10) und (3) eindosiert. Als Katalysatormenge kommt eine solche von 0,01 bis 10 Gew.-%, bezogen auf das umlaufende EGC bzw. PGC, in Frage. Diese Mengen sind abhängig von der Art des einzusetzenden Katalysators; sie sind dem Fachmann bekannt und nicht Gegenstand der vorliegenden Erfindung.

Ein Teil des eingesetzten Katalysators verläßt zusammen mit dem aufzuarbeitenden Reaktionsgemisch über (11) das Verfahren. Dieser Teil des Katalysators kann im Rahmen der Aufarbeitung des Reaktionsproduktes wiedergewonnen, bei Bedarf regeneriert und dem Reaktor (I) über (14) wieder zugesetzt werden. Für den Fall, daß ein Teil des Katalysators ausgeschleust werden muß, wird dieser ausgeschleuste Teil ergänzt durch frischen Katalysator über (14). Als Beispiel für die Regenerierung von erschöpftem Katalysator kann gemäß DE-OS 42 10 943 die Behandlung von Katalysatoren vom Typ der Gemische aus Alkalihalogeniden mit Halogeniden zweiwertiger Metalle, etwa NaBr/ZnBr₂, mit Halogenverbindungen, etwa mit Bromwasserstoff, genannt werden.

### Beispiel

In den bei 16 bar betriebenen Reaktor gemäß Fig. 1 wurden stündlich insgesamt über (1) 0,275 kg CO₂ und über (2) 0,220 kg EOX eindosiert und zwar so, daß je 0,110 kg CO₂ bzw. EOX als Gemisch durch die Leitungen (4) bis (5) flossen und 0,055 kg CO₂ dem über (10) mit einer Temperatur von 120°C zurücklaufenden Reaktionsgemisch von etwa 19,5 kg vor Eintritt in die Blasensäule (I) zugemischt (3) wurden.

Nach Ablauf der Reaktion verließen 19,940 kg Reaktionsgemisch mit einer Temperatur von ca. 135°C den Reaktor und flossen als Strom (8) in die Wärmetauscher (II) und (III). Hiernach betrug die Temperatur des Reaktionsgemisches etwa 120°C. Das Reaktionsgemisch wurde dann in den Strom (10) von ca. 19,060 kg und (11) von ca. 0,880 kg aufgeteilt. (10) kehrte, wie beschrieben, in den Reaktor zurück, während (11) in (IV) auf 1 bar entspannt wurde, wobei die gasförmige Phase (13) und die flüssige Phase (12) anfiel. (12) wurde in einen mit der Wärmemenge aus (II) betriebenen Fallfilmverdampfer geleitet und dort bei etwa 15 mbar in 0,440 kg dampfförmiges EGC und 0,440 kg Sumpf gespalten.

Nach Aufteilung dieses Sumpfes im Verhältnis 9:1 floß die größere Menge von 0,396 kg direkt und die kleinere von 0,044 kg gegebenenfalls nach einer Regenerierung als Katalysatorlösung (14) zurück in den Reaktor.

Diesem Beispiel lagen weiterhin folgende Parameter zugrunde:

| | |
|---|---|
| Katalysator | NaBr/ZnBr₂ (molar 2:1) |
| Katalysatorkonzentration im Reaktor | 0,3 Gew.-% |
| Reinheit EOX >99,5 % | |
| Reinheit CO₂ >99,9 % | |
| Art der Einbauten zur Edukteinspeisung | Rohrverteiler |
| Art der Einbauten zur Gasverteilung | Lochplatten |
| Anzahl der Einbauten zur Gasverteilung | 11 |
| Schlankheitsgrad der Blasensäule | 35 |
| Volumen der Blasensäule | 0,64 l |
| Abstand der Einbauten zur Gasverteilung (bezogen auf Blasensäulendurchmesser) | 3,5 |
| Gasgeschwindigkeit in den Bohrungen der Einbauten zur Edukteinspeisung | ca. 2,8 m/s (Durchschnitt; 2 bis 7 m/s, je nach Ort im Reaktor) |
| zur Gasverteilung und Durchmischung | |
| Der EOX-Umsatz über den gesamten Reaktor betrug 99,8 %. Die GC-Analyse des Rohcarbonates zeigte 99,4 bis 99,5 % EGC, die des Reincarbonates nach Destillation ≥ 99,6 % | |

## Patentansprüche

1. Reaktor zur kontinuierlichen Herstellung von Ethylenglykolcarbonat (EGC) bzw. Propylenglykolcarbonat (PGC) aus CO₂ und Ethylenoxid (EOX) bzw. Propylenoxid (POX) in bereits vorliegendem EGC bzw. PGC als Reaktionsmedium in Form einer Blasensäule, gekennzeichnet durch a) einen Schlankheitsgrad als Verhältnis von Höhe zu Durchmesser von 2 bis 80, bevorzugt 2 bis 50, besonders bevorzugt 5 bis 30, b) einen Zulauf für das als Reaktionsmedium dienende bereits vorliegende EGC bzw. PGC am unteren Ende der Blasensäule, c) eine oder mehrere Dosierstellen für die Edukte CO₂ und EOX bzw. POX oberhalb des Zulaufs b), d) einen Ablauf für das Reaktionsgemisch am oberen Ende der Blasensäule, e) eine an d) anschließende Aufteilungseinrichtung für das ablaufende Reaktionsgemisch zur Aufteilung in zu entnehmendes und aufzuarbeitendes Reaktionsgemisch (11) und in zu recyclisierendes Reaktionsgemisch (10) und f) eine Rückführungsleitung (10) und (3) zum Zulauf b) für das zu recyclisierende Reaktionsgemisch.

2. Verfahren zur kontinuierlichen Herstellung von Ethylenglykolcarbonat (EGC) bzw. Propylenglykolcarbonat (PGC) durch Umsetzung von Ethylenoxid (EOX) bzw. Propylenoxid (POX) mit 1,01 bis 1,5 Mol, bevorzugt 1,01 bis 1,4 Mol, besonders bevorzugt 1,01 bis 1,35 Mol CO₂ pro Mol Alkylenoxid im Temperaturbereich von 100 bis 200°C, bevorzugt 100 bis 190°C, besonders bevorzugt 110 bis 180°C und im Druckbereich von 5 bis 200 bar, bevorzugt 5 bis 80 bar, besonders bevorzugt 8 bis 60 bar im zu bildenden EGC bzw. PGC als Reaktionsmedium in einer Menge, die das 7- bis 350-fache des neu gebildeten EGC bzw. PGC beträgt, dadurch gekennzeichnet, daß zur Umsetzung ein Blasensäulenreaktor nach Anspruch 1 verwendet wird.

3. Reaktor nach Anspruch 1, gekennzeichnet durch eine kaskadierte Bauweise.

4. Reaktor nach Anspruch 1, gekennzeichnet durch Einbauten in Form von Lochböden, Rohrverteilern, Düsen, Zweistoffdüsen, Strahldüsen, geschlossenen Gasverteilern, Impingment Aerator-Elementen, Mischerelementen oder einer Kombination mehrerer von ihnen.

5. Reaktor nach Anspruch 4, gekennzeichnet durch einen Abstand zwischen zwei aufeinanderfolgenden Einbauten im Wert des 0,1- bis 10-fachen, bevorzugt 0,3- bis 8-fachen des Durchmessers der Blasensäule, durch einen freien Begasungsquerschnitt, der eine Gasgeschwindigkeit von 0,1 bis 15 m/s, bevorzugt 0,2 bis 12 m/s zuläßt, und durch Durchmesser für Bohrungen in den Einbauten im Bereich von 0,2 bis 20 mm, bevorzugt 0,5 bis 10 mm, besonders bevorzugt 0,5 bis 8 mm.

6. Reaktor nach Anspruch 1, gekennzeichnet durch einen von unten nach oben abnehmenden freien Strömungsquerschnitt der aufeinanderfolgenden Einbauten.

7. Reaktor nach Anspruch 1, gekennzeichnet durch eine Zuführungsleitung für CO₂ in die Rückführungsleitung (10) und (3) des zurückzuführenden EGC bzw. PGC und 1 bis 10, bevorzugt 2 bis 5 separate Eindosierstellen für die gemeinsame Eindosierung von CO₂ und EOX bzw. CO₂ und POX.

8. Verfahren nach Anspruch 2, dadurch gekennzeichnet, daß eine adiabatische Verfahrensweise mit einer Temperatursteigerung des Reaktionsgemisches zwischen Eingang und Ausgang um 2 bis 80°C durchgeführt wird.

9. Verfahren nach Anspruch 8, dadurch gekennzeichnet, daß die adiabatisch gespeicherte Reaktionswärme im Reaktionsgemisch zur Erzeugung von Prozeßdampf oder zur Aufarbeitung des ausgeschleusten Reaktionsgemisches auf reines EGC bzw. reines PGC eingesetzt wird, wobei sich das Reaktionsgemisch um eine Temperatur von 2 bis 80°C abkühlt und in dieser Form an den Reaktoreingang zurückgeführt wird.

## Claims

1. Reactor for the continuous preparation of ethylene glycol carbonate (EGC) or propylene glycol carbonate (PGC) from CO₂ and ethylene oxide (EOX) or propylene oxide (POX), respectively, in EGC or PGC, already present, as reaction medium in the form of a bubble column, characterized by a) a slenderness ratio as ratio of height to diameter of 2 to 80, preferably 2 to 50, particularly preferably 5 to 30, b) a feed, at the bottom end of the bubble column, for the EGC or PGC, already present serving as reaction medium, c) one or more metering sites for the starting materials CO₂ and EOX or POX above the feed b), d) an outlet for the reaction mixture at the top end of the bubble column, e) a dividing device connected to d) for the reaction mixture flowing off for dividing it into reaction mixture (11) to be taken off and worked up and reaction mixture (10) to be recycled and f) a return line (10) and (3) to the feed b) for the reaction mixture to be recycled.

2. Process for the continuous preparation of ethylene glycol carbonate (EGC) or propylene glycol carbonate (PGC) by reaction of ethylene oxide (EOX) or propylene oxide (POX), respectively, with 1.01 to 1.5 mol, preferably 1.01 to 1.4 mol, particularly preferably 1.01 to 1.35 mol, of CO₂ per mol of alkylene oxide in the temperature range from 100 to 200°C, preferably 100 to 190°C, particularly preferably 110 to 180°C and in the pressure range from 5 to 200 bar, preferably 5 to 80 bar, particularly preferably 8 to 60 bar in the EGC or PGC to be formed as reaction medium in an amount which is 7 to 350 times that of the newly formed EGC or PGC, characterized in that a bubble column reactor according to Claim 1 is used for the reaction.

3. Reactor according to Claim 1, characterized by a cascading structure.

4. Reactor according to Claim 1, characterized by internals in the form of perforated trays, pipe distributors, nozzles, two-component nozzles, jet nozzles, closed gas distributors, impingement aerator elements, mixer elements or a combination of a plurality of these.

5. Reactor according to Claim 4, characterized by a spacing between two sequentially following internals of a value of 0.1 to 10 times, preferably 0.3 to 8 times that of the diameter of the bubble column, by a free gas introduction cross-section which permits a gas velocity of 0.1 to 15 m/s, preferably 0.2 to 12 m/s, and by diameters for boreholes in the internals in the range from 0.2 to 20 mm, preferably 0.5 to 10 mm, particularly preferably 0.5 to 8 mm.

6. Reactor according to Claim 1, characterized by a free flow cross-section of the sequentially following internals decreasing from bottom to top.

7. Reactor according to Claim 1, characterized by a feed line for CO₂ into the return line (10) and (3) of the returning EGC or PGC and 1 to 10, preferably 2 to 5, separate addition sites for the joint addition of CO₂ and EOX or CO₂ and POX.

8. Process according to Claim 2, characterized in that an adiabatic procedure is carried out with a temperature increase in the reaction mixture between inlet and outlet of 2 to 80°C.

9. Process according to Claim 8, characterized in that the adiabatically stored heat of reaction in the reaction mixture is used to generate process steam or to work up the ejected reaction mixture to give pure EGC or pure PGC, the reaction mixture cooling by a temperature of 2 to 80°C and being returned in this form to the reactor entrance.

## Revendications

1. Réacteur pour la préparation en continu de carbonate d'éthylène glycol (CEG) ou de carbonate de propylène glycol (CPG) à partir de CO₂ et d'oxyde d'éthylène (EOX) ou d'oxyde de propylène (POX) dans le CEG ou le CPG déjà présent et qui sert de milieu de réaction, lequel réacteur présente la forme d'une colonne à bulles, caractérisé par a) un rapport d'allongement, représenté par le rapport entre la hauteur et le diamètre, de 2 à 80, de préférence de 2 à 50 et de manière particulièrement préférable de 5 à 30, b) une amenée pour le CEG ou le PGC déjà présent et servant de milieu de réaction située à l'extrémité inférieure de la colonne à bulles, c) un ou plusieurs emplacements d'introduction des réactifs CO₂ et EOX ou POX, situés au-dessus de l'amenée b), d) une évacuation du mélange de réaction située à l'extrémité supérieure de la colonne à bulles, e) un dispositif de répartition, raccordé à d), pour diviser le mélange de réaction sortant en un mélange de réaction (11) à extraire et à transformer et en un mélange de réaction (10) à recycler, et f) un circuit de retour (10) et (3) vers l'amenée b), pour le mélange de réaction à recycler.

2. Procédé pour la préparation en continu de carbonate d'éthylène glycol (CEG) ou de carbonate de propylène glycol (CPG) par réaction d'oxyde d'éthylène (EOX) ou d'oxyde de propylène (POX) avec 1,01 à 1,5 mole, de préférence 1,01 à 1,4 mole, et de manière particulièrement préférable 1,01 à 1,35 mole de CO₂ par mole d'oxyde d'alkylène, dans la plage des températures comprises entre 100 et 200° C, de préférence entre 100 et 190° C, et de manière particulièrement préférable entre 110 et 180° C et dans la plage des pressions de 5 à 200 bars, de préférence de 5 à 80 bars et de manière particulièrement préférable de 8 à 60 bars dans le CEG ou le CPG à former qui sert de milieu de réaction et est présent en une quantité qui représente de 7 à 350 fois le CEG ou le CPG nouvellement formé, caractérisé en ce que pour la conversion, on utilise un réacteur à colonne à bulles selon la revendication 1.

3. Réacteur selon la revendication 1, caractérisé en ce qu'il présente une structure en cascade.

4. Réacteur selon la revendication 1, caractérisé par des garnitures qui se présentent sous la forme de plateaux perforés, de répartiteurs tubulaires, de buses, de buses à deux jets, de buses de projection, de répartiteurs de gaz fermés et d'éléments d'aération à aspersion de gouttes, d'éléments mélangeurs ou d'une combinaison de plusieurs de ceux-ci.

5. Réacteur selon la revendication 4, caractérisé en ce qu'une distance entre deux garnitures successives vaut de 0,1 à 10 fois, et de préférence de 0,3 à 8 fois le diamètre de la colonne à bulles, en ce qu'une section libre d'injection de gaz permet une vitesse de gaz de 0,15 à 15 m/s, et de préférence de 0,2 à 12 m/s, et en ce qu'un diamètre des alésages des garnitures est compris dans la plage de 0,2 à 20 mm, de préférence de 0,5 à 10 mm et de manière particulièrement préférable de 0,5 à 8 mm.

6. Réacteur selon la revendication 1, caractérisé en ce que la section transversale libre d'écoulement des garnitures successives diminue du bas vers le haut.

7. Réacteur selon la revendication 1, caractérisé par une conduite d'amenée de CO₂ dans le circuit de retour (10) et (3) du CEG ou du PGC à recirculer, et par 1 à 10, de préférence 2 à 5 emplacements d'introduction dosée distincts, pour l'introduction dosée commune de CO₂ et d'EOX ou de CO₂ et de POX.

8. Procédé selon la revendication 2, caractérisé en ce qu'il est mis en oeuvre de manière à obtenir une augmentation de température de 2 à 80° C entre l'entrée et la sortie du mélange de réaction.

9. Procédé selon la revendication 8, caractérisé en ce que la chaleur de réaction emmagasinée adiabatiquement dans le mélange de réaction est utilisée pour la création de vapeur de processus ou pour la transformation en CEG pur ou en PGC pur du mélange de réaction extrait, le mélange de réaction se refroidissant d'une température de 2 à 80° C et étant renvoyé sous cette forme à l'entrée du réacteur.
